# EUROPEAN PATENT SPECIFICATION

(11) **EP 1 388 284 B1**
(45) Date of publication and mention of the grant of the patent: **21.02.2007**
(21) Application number: 03017045.0
(22) Date of filing: 28.07.2003
(51) Int. Cl.: A01M 19/00

(54) **Structure for environmentally-safe disinfestation using mobile chambers**
Struktur zur umweltschönenden Entwesung mittels mobilen Kammern
Structure pour la désinfestation respectueuse de l'environnement utilisant des chambres mobiles

(30) Priority: 05.08.2002 IT MI20021769
(43) Date of publication of application: 11.02.2004
(73) Proprietor: ISOLCELL ITALIA S.P.A., I-39055 Laives (BZ) (IT)
(72) Inventor: Pruneri, Dario, 39100 Bolzano (IT); Villa, Ivano, 39100 Bolzano (IT)
(74) Representative: Forattini, Amelia

(56) References cited:
- DE-A- 4 429 850
- DE-A- 19 540 564
- FR-A- 2 643 232

## Description

The present invention relates to a structure for environmentally-safe disinfestation using mobile chambers.

The structure according to the invention is particularly useful for mobile environmentally-safe disinfestation of works of art, items of historical and artistic importance, and foodstuffs.

Disinfestation systems based on the production and maintenance of an asphyxial atmosphere, i.e., an atmosphere with an extremely low concentration of oxygen, inside a mobile gas-tight disinfestation chamber usually constituted by a tent erected around the item to be disinfested, are known.

ln prior art disinfestation systems, the reduction in the percentage of oxygen in the tent is obtained by diluting the internal atmosphere by introducing a mixture having a low oxygen content.

DE-A-19540564 discloses a storage device comprising a gas-tight case hung from a support frame. The case can be filled with a processing gas.

DE-A-4429850 discloses a process for killing pests using an inert gas, such as nitrogen, carbon dioxide, cyan or a mixture of these gases. The inert gas is warmed and during heating, the inert gas atmosphere is supplied with moisture to maintain the hygroscopic equilibrium in the component. At the end of the process, the temperature is returned to room temperature.

FR-A-2643232 discloses a method for preserving and protecting a very large number of articles of any type against oxygen in the air, humidity, insects, rodents, aging, self-explosion, fires, etc. The articles to be preserved are placed in hermetically closed preservation chambers from which air has been sucked out as much as possible, then replaced by any inert gas coming from a gas tank joined to the preservation chamber. This inert gas remains mixed with the articles throughout the preservation. ln order to add or withdraw articles, the inert gas is first withdrawn from the preservation chamber by means of a pump and compressed into the same above mentioned tank, then air is let in order to replace the gas, after which the chamber is opened.

The above described prior art methods and devices are not suitable for use with mobile chambers and cannot effectively be used with objects of considerable size and that cannot be moved from their location.

The aim of the present invention is to provide a disinfestation structure improved with respect to the prior art.

An object of the invention is to provide a disinfestation structure that allows to optimize the performance of the equipment and accordingly allows a significant reduction in the time required by the machine to reach the low oxygen levels required for correct execution of the disinfestation process.

A further object of the invention is to provide a structure that can work in a controlled and automatic manner, so as to allow operation in a continuous cycle without intervention on the part of operators.

A further object is to provide a structure in which control of the temperature and relative humidity inside the tent allows to increase and optimize the lethal effect of the atmosphere with low oxygen content on the infesting agents, allowing to reduce the duration of the disinfestation treatment.

A further object is to provide a structure that has a low cost and is extremely reliable.

This aim, these objects and others that will become better apparent hereinafter are achieved by a structure for environmentally-safe disinfestation using mobile chambers, as claimed in the appended claims.

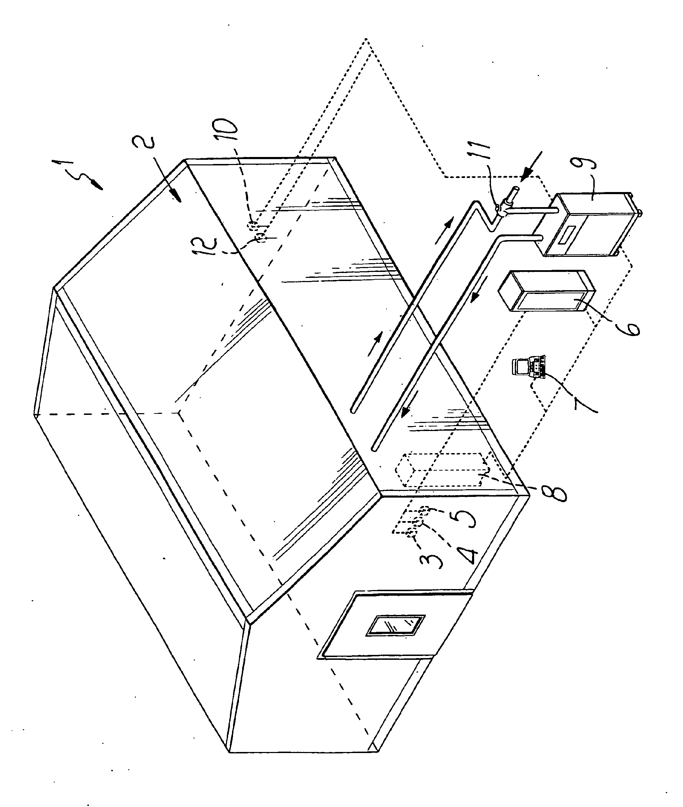

Further characteristics and advantages will become better apparent from the description of preferred but not exclusive embodiments of the invention, illustrated only by way of non-limitative example in the accompanying drawing, wherein the only figure is a schematic perspective view of a mobile structure for disinfestation according to the invention.

With reference to the figure, the mobile structure according to the invention, generally designated by the reference numeral 1, comprises a tent 2, which is made hermetic and in which the levels of residual oxygen, temperature and relative humidity are sensed by means of sensors respectively designated by the reference numerals 3, 4 and 5.

These data are collected, stored and processed by a computerized analysis system, which has the task of monitoring and controlling the entire disinfestation process.

The computerized analysis system is preferably constituted by a control PLC 6, which is connected to a personal computer 7 for storing the data and setting the operating parameters.

The temperature inside the tent is maintained by a heating and ventilation system 8, which in this case is located inside the tent; the system is controlled by the analysis system.

Likewise, the constancy of the relative humidity is ensured by a humidification system that is connected to the tent and is controlled by the computerized analysis system.

A nitrogen generator or, alternatively, an oxygen absorber, designated by the reference numeral 9, connected in a closed circuit and also controlled by the computerized control system, has the task of reducing and then keeping constant the oxygen level.

The volume of the oxygen extracted from the tent is restored automatically by way of a system for controlling the volume of the tent.

ln practice, whereas the mixture generated by the oxygen absorber is conveyed into the tent at all times, a PLC, functionally connected to the absorber, cyclically switches the intake of the air supply between the inside and the outside of the tent.

The PLC is controlled by the change of state of a microswitch 10, which is connected to one of the walls of the tent.

Depending on whether the tent contracts or expands, the movement of the wall switches the state of the microswitch and this signal activates the switching of the state of an intake valve 11 of the absorber.

The absorber therefore cyclically reduces and increases the volume of the atmosphere contained in the tent, depending on whether it generates the mixture with low oxygen content by extracting it from the tent itself or from the outside.

With this system, the absorber automatically maintains the volume of the tent within a fixed interval that is set by the stroke of the microswitch 10.

A second safety microswitch 12 controls the enabling of the operation of the absorber, preventing the machine from continuing to operate if the volume of the tent varies too far from the normal volume.

ln practice it has been found that the invention achieves the intended aim and objects, having provided a disinfestation structure in which the reduction in the percentage of oxygen in the tent occurs by "extracting" the oxygen from the atmosphere of the tent itself, since the oxygen absorber 9 operates in a closed circuit instead of by diluting the internal atmosphere by introducing an oxygen-poor mixture, as occurs in current systems.

This leads to an optimization of the performance of the absorber or generator that is used and accordingly to a significant reduction in the time required by the machine to reach the low levels of oxygen required for the correct execution of the disinfestation process.

By extracting the oxygen from the tent, it becomes necessary to replenish the volume inside the tent occupied by the extracted oxygen in a controlled and automatic way, so as to allow the operation of the machine in a continuous cycle without reducing the volume of the tent during the oxygen reduction step.

The system according to the present invention is cheap and extremely reliable.

Control of the temperature and relative humidity inside the tent allow to increase and optimize the lethal effect of the oxygen-poor atmosphere on infesting agents, allowing to reduce the duration of the disinfestation treatment.

It is also possible to control the mechanical stresses affecting the treated items caused by any sudden gradients in temperature or relative humidity inside the tent.

Control of all the variables of the disinfestation process, of temperature, relative humidity and residual oxygen ensures correct and reliable execution of the disinfestation process.

The structure according to the invention is susceptible of numerous modifications and variations, within the scope of the appended claims.

## Claims

1. A structure for environmentally-safe disinfestation using mobile chambers, comprising a hermetic mobile chamber that is suitable to contain one or more items to be disinfested and is functionally connected to a means for controlling the atmosphere inside said chamber, **characterized in that** it comprises a means for controlling the internal volume of said chamber by detecting a variation in pressure and by integrating the volume of the mixture of gases contained in the chamber in order to maintain a substantially constant volume.

2. The structure according to claim 1, **characterized in that** said atmosphere control means comprises an oxygen absorber.

3. The structure according to claim 1, **characterized in that** said atmosphere control means comprises a nitrogen generator.

4. The structure according to one or more of the preceding claims, **characterized in that** said mobile chamber is constituted by a tent.

5. The structure according to one or more of the preceding claims, **characterized in that** it comprises, inside said mobile chamber, sensors adapted to detect the levels of residual oxygen, temperature and relative humidity inside said chamber.

6. The structure according to one or more of the preceding claims, **characterized in that** it comprises a computerized analysis system entrusted with monitoring and controlling the entire disinfestation process and with collecting and storing said values of residual oxygen, temperature and relative humidity sensed inside said chamber.

7. The structure according to one or more of the preceding claims, **characterized in that** said computerized analysis system comprises a control PLC that is connected to a personal computer for storing the data and setting the operating parameters.

8. The structure according to one or more of the preceding claims and claim 4, **characterized in that i**t comprises a heating and ventilation system that is controlled by the analysis system and is suitable to control the temperature inside the tent.

9. The structure according to one or more of the preceding claims and claim 4, **characterized in that** it comprises a humidification system that is connected to the tent, is controlled by the computerized analysis system and is suitable to ensure the constancy of the relative humidity.

10. The structure according to one or more of the preceding claims, **characterized in that** said oxygen absorber is connected in a closed circuit, is always controlled by the computerized control system and is entrusted with reducing the oxygen level and then keeping it constant.

11. The structure according to one or more of the preceding claims and claim 4, **characterized in that** it comprises a PLC that is suitable to switch cyclically the intake of the air supply between the inside and the outside of said tent, whereas the mixture generated by the oxygen absorber is always conveyed into the tent.

12. The structure according to claim 11, **characterized in that** said PLC is controlled by the variation of the state of a microswitch that is connected to one of the walls of the tent.

13. The structure according to one or more of the preceding claims and claim 4, **characterized in that** depending on whether the tent contracts or expands, the movement of the wall causes the switching of the state of said microswitch, and this signal activates the switching of the state of an intake valve of the absorber.

14. The structure according to one or more of the preceding claims and claim 4, **characterized in that** said absorber cyclically reduces and increases the volume of the atmosphere contained in the tent, depending on whether it generates the oxygen-poor mixture by extracting it from said tent or from the outside.

15. The structure according to one or more of the preceding claims and claim 4, **characterized in that** it comprises a second safety microswitch that controls the enabling of the operation of the absorber, preventing the machine from continuing to operate if the volume of the tent varies excessively with respect to the normal volume.

## Patentansprüche

1. Anordnung zur umweltverträglichen Entwesung unter Verwendung von mobilen Kammern, aufweisend eine hermetische mobile Kammer, die zur Aufnahme von einem oder mehreren zu entwesenden Objekten geeignet ist und die funktionsbedingt mit einem Mittel zur Regelung der Atmosphäre in der Kammer verbunden ist, **dadurch gekennzeichnet, dass** die Anordnung ein Mittel zur Regelung des Innenvolumens der Kammer durch Erfassung einer Druckschwankung und durch Volumenintegration der in der Kammer enthaltenen Mischung aus Gasen aufweist, um ein im Wesentlichen konstantes Volumen zu erhalten.

2. Anordnung nach Anspruch 1, **dadurch gekennzeichnet, dass** das Regelungsmittel der Atmosphäre einen Sauerstoffabsorber aufweist.

3. Anordnung nach Anspruch 1, **dadurch gekennzeichnet, dass** das Regelungsmittel der Atmosphäre einen Stickstofferzeuger aufweist.

4. Anordnung nach einem oder mehreren der vorhergehenden Ansprüche, **dadurch gekennzeichnet, dass** die mobile Kammer durch ein Zelt gebildet ist.

5. Anordnung nach einem oder mehreren der vorhergehenden Ansprüche, **dadurch gekennzeichnet, dass** die Anordnung in der mobilen Kammer Sensoren aufweist, die zur Erfassung der Pegel des Restsauerstoffs, der Temperatur und der relativen Luftfeuchtigkeit in der Kammer geeignet sind.

6. Anordnung nach einem oder mehreren der vorhergehenden Ansprüche, **dadurch gekennzeichnet, dass** die Anordnung ein computergesteuertes Analysesystem aufweist, das mit der Überwachung und Regelung des gesamten Entwesungsprozesses und mit dem Sammeln und Speichern der Werte des Restsauerstoffs, der Temperatur und der relativen Luftfeuchtigkeit in der Kammer beauftragt ist.

7. Anordnung nach einem oder mehreren der vorhergehenden Ansprüche, **dadurch gekennzeichnet, dass** das computergesteuerte Analysesystem einen Regelungs-PLC aufweist, der zur Speicherung der Daten und Einstellung der Betriebsparameter mit einem Personalcomputer verbunden ist.

8. Anordnung nach einem oder mehreren der vorhergehenden Ansprüche und Anspruch 4, **dadurch gekennzeichnet, dass** die Anordnung ein Heizungs- und Belüftungssystem aufweist, das durch das Analysesystem geregelt wird und das zur Regelung der Temperatur in dem Zelt geeignet ist.

9. Anordnung nach einem oder mehreren der vorhergehenden Ansprüche und Anspruch 4, **dadurch gekennzeichnet, dass** die Anordnung ein mit dem Zelt verbundenes Feuchtegraderhöhungssystem aufweist, das durch das computergesteuerte Analysesystem geregelt wird und das zur Gewährleistung der Konstanz der relativen Luftfeuchtigkeit geeignet ist.

10. Anordnung nach einem oder mehreren der vorhergehenden Ansprüche, **dadurch gekennzeichnet, dass** der an einen geschlossenen Kreislauf angeschlossene Sauerstoffabsorber ständig durch das computergesteuerte Regelungssystem geregelt wird und mit der Reduzierung des Sauerstoffpegels und der anschließenden Konstanthaltung desselben beauftragt ist.

11. Anordnung nach einem oder mehreren der vorhergehenden Ansprüche, **dadurch gekennzeichnet, dass** die Anordnung einen PLC aufweist, der zum periodischen Umschalten des Einlasses der Luftzuführung zwischen dem Inneren und dem Äußeren des Zeltes geeignet ist, wobei die durch den Sauerstoffabsorber erzeugte Mischung ständig in das Zelt gefördert wird.

12. Anordnung nach Anspruch 11, **dadurch gekennzeichnet, dass** der PLC durch die Zustandsschwankungen eines Mikroschalters gesteuert wird, der mit einer der Wände des Zeltes verbunden ist.

13. Anordnung nach einem oder mehreren der vorhergehenden Ansprüche, **dadurch gekennzeichnet, dass** die Bewegung der Wand das Umschalten des Zustandes des Mikroschalters in Abhängigkeit davon bedingt, ob sich das Zelt entweder zusammenzieht oder ausdehnt, und dieses Signal aktiviert das Umschalten des Zustandes eines Einlassventils des Absorbers.

14. Anordnung nach einem oder mehreren der vorhergehenden Ansprüche, **dadurch gekennzeichnet, dass** der Absorber das in dem Zelt enthaltene Volumen der Atmosphäre periodisch verkleinert und vergrößert, abhängig davon, ob der Absorber die sauerstoffarme Mischung entweder aus dem Zelt oder von dem Äußeren entnimmt.

15. Anordnung nach einem oder mehreren der vorhergehenden Ansprüche, **dadurch gekennzeichnet, dass** die Anordnung einen zweiten Sicherheitsmikroschalter aufweist, der die Freigabe des Betriebs des Absorbers steuert, um zu verhindern, dass die Vorrichtung den Betrieb fortsetzt, wenn das Volumen des Zeltes mit Bezug auf das normale Volumen übermäßig schwankt.

## Revendications

1. Structure pour désinfestation respectueuse de l'environnement en utilisant des chambres mobiles, comprenant une chambre mobile hermétique qui est appropriée pour contenir un ou plusieurs articles à désinfester et qui est raccordée fonctionnellement à un moyen pour commander l'atmosphère à l'intérieur de ladite chambre, **caractérisé en ce qu'**elle comprend un moyen pour commander le volume interne de ladite chambre en détectant une variation de pression et en intégrant le volume du mélange de gaz contenus dans la chambre pour maintenir un volume sensiblement constant.

2. Structure selon la revendication 1, **caractérisée en ce que** ledit moyen de commande de l'atmosphère comprend un absorbeur d'oxygène.

3. Structure selon la revendication 1, **caractérisée en ce que** ledit moyen de commande de l'atmosphère comprend un générateur d'azote.

4. Structure selon une ou plusieurs des revendications précédentes, **caractérisée en ce que** ladite chambre mobile est constituée d'une tente.

5. Structure selon une ou plusieurs des revendications précédentes, **caractérisée en ce qu'**elle comprend, à l'intérieur de ladite chambre mobile, des capteurs adaptés pour détecter les niveaux d'oxygène résiduel, de température et d'humidité relative à l'intérieur de ladite chambre.

6. Structure selon une ou plusieurs des revendications précédentes, **caractérisée en ce qu'**elle comprend un système d'analyse informatisé chargé de surveiller et de commander tout le processus de désinfestation et de collecter et de stocker lesdites valeurs d'oxygène résiduel, de température et d'humidité relative détectées à l'intérieur de ladite chambre.

7. Structure selon une ou plusieurs des revendications précédentes, **caractérisée en ce que** ledit système d'analyse informatisé comprend un PLC de commande qui est raccordé à un ordinateur personnel pour stocker les données et régler les paramètres de fonctionnement.

8. Structure selon une ou plusieurs des revendications précédentes et la revendication 4, **caractérisée en ce qu'**elle comprend un système de chauffage et de ventilation qui est commandé par le système d'analyse et est approprié pour commander la température à l'intérieur de la tente.

9. Structure selon une ou plusieurs des revendications précédentes et la revendication 4, **caractérisée en ce qu'**elle comprend un système d'humidification qui est raccordé à la tente, commandé par ledit système d'analyse informatisé et approprié pour assurer la constance de l'humidité relative.

10. Structure selon une ou plusieurs des revendications précédentes, **caractérisée en ce que** ledit absorbeur d'oxygène est raccordé en circuit fermé, est toujours commandé par le système de commande informatisé et est chargé de réduire le niveau d'oxygène et ensuite de le maintenir constant.

11. Structure selon une ou plusieurs des revendications précédentes et la revendication 4, **caractérisée en ce qu'**elle comprend un PLC qui convient pour commuter de manière cyclique l'admission de l'alimentation en air entre l'intérieur et l'extérieur de ladite tente, tandis que le mélange généré par l'absorbeur d'oxygène est toujours acheminé dans la tente.

12. Structure selon la revendication 11, **caractérisée en ce que** ledit PLC est commandé par la variation de l'état d'un microcommutateur qui est raccordé à l'une des parois de la tente.

13. Structure selon une ou plusieurs des revendications précédentes et la revendication 4, **caractérisée en ce que**, selon que la tente se contracte ou se dilate, le mouvement de la paroi entraîne la commutation de l'état dudit microcommutateur et ce signal active la commutation de l'état d'une soupape d'admission de l'absorbeur.

14. Structure selon une ou plusieurs des revendications précédentes et la revendication 4, **caractérisée en ce que** ledit absorbeur réduit et augmente de manière cyclique le volume de l'atmosphère contenu dans la tente, selon qu'elle génère le mélange pauvre en oxygène par extraction de celui-ci de la tente ou de l'extérieur.

15. Structure selon une ou plusieurs des revendications précédentes et la revendication 4, **caractérisée en ce qu'**elle comprend un second microcommutateur de sécurité qui commande l'activation du fonctionnement de l'absorbeur, empêchant la machine de continuer à fonctionner si le volume de la tente varie de manière excessive par rapport au volume normal.
